# EUROPEAN PATENT SPECIFICATION

(11) **EP 3 873 360 B1**
(45) Date of publication and mention of the grant of the patent: **19.03.2025**
(21) Application number: 19800953.2
(22) Date of filing: 31.10.2019
(51) Int. Cl.: A61B 17/3207, A61B 17/00

(54) **ATHERECTOMY DEVICES INCLUDING PRE-SHAPED AND CURVED DISTAL PORTIONS**
ATHEREKTOMIEVORRICHTUNGEN MIT VORGEFORMTEN UND GEKRÜMMTEN DISTALEN ABSCHNITTEN
DISPOSITIFS D'ATHÉRECTOMIE COMPRENANT DES PARTIES DISTALES PRÉFORMÉES ET INCURVÉES

(30) Priority: 01.11.2018 US 201862754228 P
(43) Date of publication of application: 08.09.2021
(73) Proprietor: Koninklijke Philips N.V., 5656 AG Eindhoven (NL)
(72) Inventor: ESCUDERO, Paul, Q, 5656 AE Eindhoven (NL); ROWE, Douglas, 5656 AE Eindhoven (NL); POMBO, August, Christopher, 5656 AE Eindhoven (NL)
(74) Representative: Philips Intellectual Property & Standards
(86) International application number: PCT/EP2019/079876
(87) International publication number: WO 2020/089415

(56) References cited:
- US-A1- 2005 209 610
- US-A1- 2013 103 062
- US-A1- 2016 242 808
- US-B1- 6 497 711

## Description

### FIELD OF THE DISCLOSURE

The devices and methods described herein generally relate to treatment of occluded body lumens, such as the removal of occlusive material from a blood vessel or other body parts.

### BACKGROUND

Peripheral and interventional cardiology is a medical specialty that relates to treatment of various forms of cardiovascular disease, including coronary artery disease and peripheral vascular disease. Coronary artery disease and peripheral vascular disease can arise due to the narrowing of the arteries by atherosclerosis (also called arteriosclerosis). Coronary artery disease generally affects arteries of the heart-arteries that carry blood to cardiac muscles and surrounding tissue. Peripheral vascular disease refers to various diseases of the vascular system outside the heart and brain, which carries blood, for example, to the legs.

Atherosclerosis commonly affects the medium and large arteries, and may occur when fat, cholesterol, and other substances build up on the walls of arteries and form fleshy or hard/calcified structures called plaques/lesions. As plaque forms within an arterial wall, the artery may narrow and become less flexible, which may make it more difficult for blood to flow therethrough. In the peripheral arteries, the plaque is typically not localized, but can extend in length along the axis of the artery for as much as 10 mm or more (in some instance up to 400 mm or more).

Pieces of plaque can break off and move through the affected artery to smaller blood vessels, which may in some instances block them and may result in tissue damage or tissue death (embolization). In some cases, the atherosclerotic plaque may be associated with a weakening of the wall of the affected artery, which can lead to an aneurysm. Minimally invasive surgeries may be performed to remove plaque from arteries in an effort to alleviate or help prevent the complications of atherosclerosis.

A number of interventional surgical methodologies may be used to treat atherosclerosis. In balloon angioplasty, for example, a physician may advance a collapsed, intravascular balloon catheter into a narrowed artery, and may inflate the balloon to macerate and/or displace plaque against the vessel wall. A successful angioplasty may help reopen the artery and allow for improved blood flow. Often, balloon angioplasty is performed in conjunction with the placement of a stent or scaffold structure within the artery to help minimize re-narrowing of the artery. Balloon angioplasty, however, can stretch the artery and induce scar tissue formation, while the placement of a stent can cut arterial tissue and also induce scar tissue formation. Scar tissue formation may lead to restenosis of the artery. In some instances, balloon angioplasty can also rip the vessel wall.

Atherectomy is another treatment methodology for atherosclerosis, and involves the use of an intravascular device to mechanically remove (that is, debulk) plaque from the wall of the artery. Atherectomy devices may allow for the removal of plaque from the wall of an artery, reducing the risk of stretching, cutter, or dissecting the arterial wall and causing tissue damage that leads to restenosis. In some instances, atherectomy may be used to treat restenosis by removing scar tissue.

Some atherectomy devices suffer from structural and performance limitations. For example, atherectomy devices with rotating burrs (for example, the Diamondback 360^{®} PAD System, from Cardiovascular Systems, Inc.) generally are not configured to capture particles that are released as the burr grinds/sands tissue, which may result in diminished downstream blood flow resulting from particle residue. Additionally, these rotating burrs may cause hemolysis, and are generally limited as an adjunct therapy to angioplasty.

Other atherectomy devices, such as the JETSTREAM G3^{®} System, from Pathway Medical Technologies, include expandable cutters with foldable/movable cutter wings and vacuum-driven aspiration supplied via a vacuum pump, which may cause the artery to collapse on to the cutter and perforate the arterial wall. Other atherectomy systems may include a side window eccentric cutter and distal nosecone which receives material from the cutter. Because the nosecone can only hold a limited volume of plaque, a surgeon may need to repeatedly withdraw the cutter and flush plaque and other material from the nosecone.

Some atherectomy devices are reconfigurable to permit cutters to oppose (that is, at least partially face toward) blood vessel walls. Upon sweeping the cutter within a blood vessel, the cutter can treat a relatively large area of plaque and thereby provide relatively high luminal gain. However, such atherectomy devices are typically relatively complex due to the presence of actuation mechanisms for reconfiguring the devices (for example, one or more pull wires), and such devices are difficult to manufacture in relatively small sizes.

US 2016/242808 A1 relates to apparatus for cutting and removing occlusive material with imaging capabilities. The atherectomy apparatus comprises a first catheter, a second catheter, and a cutter assembly attached to the first catheter. The first catheter is moveable relative to the second catheter to move a distal portion of the atherectomy apparatus between an undeflected configuration and a deflected configuration, wherein the deflected distal portion comprises a double curve having a first proximal curve and a second distal curve.

US 2005/209610 A1 relates to a tissue removal kit or assembly, which comprises a cannula and a tissue removal probe axially slidable within the cannula.

US 6,949,711 B1 relates to an atherectomy device having the ability to create variably sized lumens in a vessel.

Accordingly, it is desirable to provide improved atherectomy devices**.**

### SUMMARY

The invention is defined by the claims.

The present disclosure presents an atherectomy device that includes a handle configured to be manipulated by a user. The atherectomy device further includes a catheter, and the catheter includes an outer sheath. The outer sheath includes a proximal portion coupled to and extending distally relative to the handle, and the proximal portion defines a longitudinal axis of the catheter. The outer sheath also includes a distal portion coupled to and extending distally relative to the proximal portion, and the distal portion normally has a curved configuration and is offset from the longitudinal axis. The distal portion comprises a pattern of slits and non-slit portions or a pattern of kerfs and non-kerf portions. The catheter further includes a drive shaft carried within and rotatable relative to the outer sheath. The catheter further includes a cutter assembly coupled to and extending distally relative to the distal portion of the outer sheath. The cutter assembly includes a cutter that is translatably fixed relative to the outer sheath. The cutter is coupled to the drive shaft and rotates about a cutter rotation axis upon rotation of the drive shaft relative to the outer sheath. The cutter rotation axis is normally disposed at an acute angle relative to the longitudinal axis.

The curved configuration includes a proximal curve and distal curve, wherein the proximal curve bends away from the longitudinal axis and the distal curve bends toward the longitudinal axis.

The catheter may be rotatable about a catheter rotation axis relative to the handle.

The catheter rotation axis may be collinear with the longitudinal axis.

The pattern may be a first pattern, the proximal portion comprises a second pattern of slits and non-slit portions, and the second pattern is different than the first pattern.

The acute angle may be about 45 degrees.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (for example, X₁ and X₂) as well as a combination of elements selected from two or more classes (for example, Y₁ and Zₒ).

The term "a" or "an" entity refers to one or more of that entity. As such, the terms "a" (or "an"), "one or more" and "at least one" may be used interchangeably herein. It is also to be noted that the terms "comprising", "including", and "having" may be used interchangeably.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure may be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
FIG. 1 is a side view of an atherectomy system according to an embodiment of the present disclosure.
FIG. 2A is a detail side view of a distal portion of the atherectomy system of FIG. 1.
FIG. 2B is a detail perspective view of the distal portion of the atherectomy system of FIG. 1.
FIG. 2C is a detail transverse sectional view of the distal portion of the atherectomy system along line 2C-2C of FIG. 2A.
FIG. 3A is a side view of a distal portion of an outer sheath of the atherectomy system of FIG. 1 in a normal configuration.
FIG. 3B is a side view of the distal portion of the outer sheath of FIG. 3A in a deflected configuration.
FIG. 4A is a side view of a proximal portion of the outer sheath of the atherectomy system of FIG. 1.
FIG. 4B is an end view of the proximal portion of the outer sheath of FIG. 4A.
FIG. 5 illustrates a catheter of the atherectomy system of FIG. 1 being passively deflected to a relatively straight configuration while navigating the vasculature of a subject.
FIG. 6 illustrates the catheter of the atherectomy system of FIG. 1 occupying a curved configuration and the cutter assembly being disposed near plaque within the vasculature of a subject.
FIG. 7 illustrates the catheter of the atherectomy system of FIG. 1 occupying a curved configuration and a cutter assembly removing plaque from the vasculature of a subject.

It should be understood that the drawings are not necessarily to scale. In certain instances, details that are not necessary for an understanding of the disclosure or that render other details difficult to perceive may have been omitted. It should be understood, of course, that the disclosure is not necessarily limited to the particular embodiments illustrated herein.

### DETAILED DESCRIPTION

Before any embodiments of the disclosure are explained in detail, it is to be understood that the disclosure is not limited in its application to the details of construction and the arrangement of components set forth in the following description or illustrated in the following drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

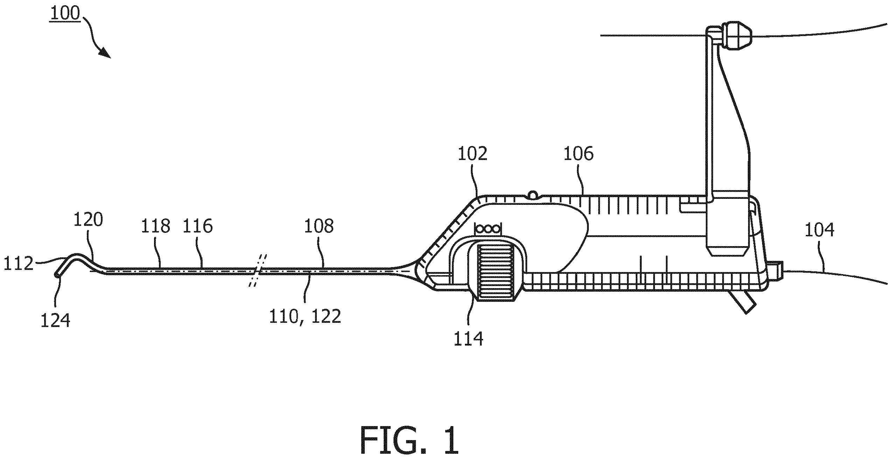

The present disclosure relates generally to devices, systems, and methods for mechanical atherectomy. Referring to FIG. 1, there is shown an exemplary embodiment of the atherectomy systems described here. The atherectomy system 100 includes an intravascular atherectomy device 102 and a guide wire 104 over which the atherectomy device 102 may be deployed. In some embodiments, the guide wire 104 is silicon-coated or non-coated (bare), or otherwise free of a PTFE coating. Atherectomy systems according to some embodiments of the present disclosure comprise a guide wire that includes a PTFE coating, or atherectomy systems according to some embodiments of the present disclosure lack a guide wire.

With continued reference to FIG. 1, the atherectomy device 102 generally includes a handle 106 and a catheter 108. The handle 106 is configured to be grasped and manipulated by a user (for example, a medical professional) during an atherectomy procedure. The catheter 108 is coupled to and extends distally relative to the handle 106. The catheter 108 is configured to be positioned in the vasculature of a subject (for example, a patient) during an atherectomy procedure to facilitate removal of plaque therefrom. In some embodiments, the catheter 108 is selectively rotatable about a catheter rotation axis 110 relative to the handle 106 to facilitate appropriately positioning and or "sweeping" a distal portion 112 of the catheter 108 during an atherectomy procedure. In some embodiments and as illustrated, the handle 106 carries a rotatable knob or dial 114 for selectively rotating the catheter 108 relative to the handle 106.

With further reference to FIG. 1, the catheter 108 includes an outer sheath 116 having a proximal portion 118 and a distal portion 120. The proximal portion 118 is coupled to and extends distally relative to the handle 106. The proximal portion 118 is an elongated component and defines a longitudinal axis 122 of the catheter 108. In some embodiments and as illustrated, the longitudinal axis 122 is collinear with a catheter rotation axis 110. In other embodiments, the longitudinal axis 122 is non-collinear with a catheter rotation axis 110. In some embodiments, the distal portion 120 mechanically couples to the proximal portion 118 (for example, via welding or the like). In other embodiments, the distal portion 120 integrally couples to, or is monolithically formed with, the proximal portion 118. The distal portion 120 extends distally relative to the proximal portion 118 and, as described in further detail below, normally has a curved configuration and is offset from the longitudinal axis 122. The catheter 108 further includes a cutter assembly 124 that is coupled to and extends distally relative to the distal portion 120 of the outer sheath 116.

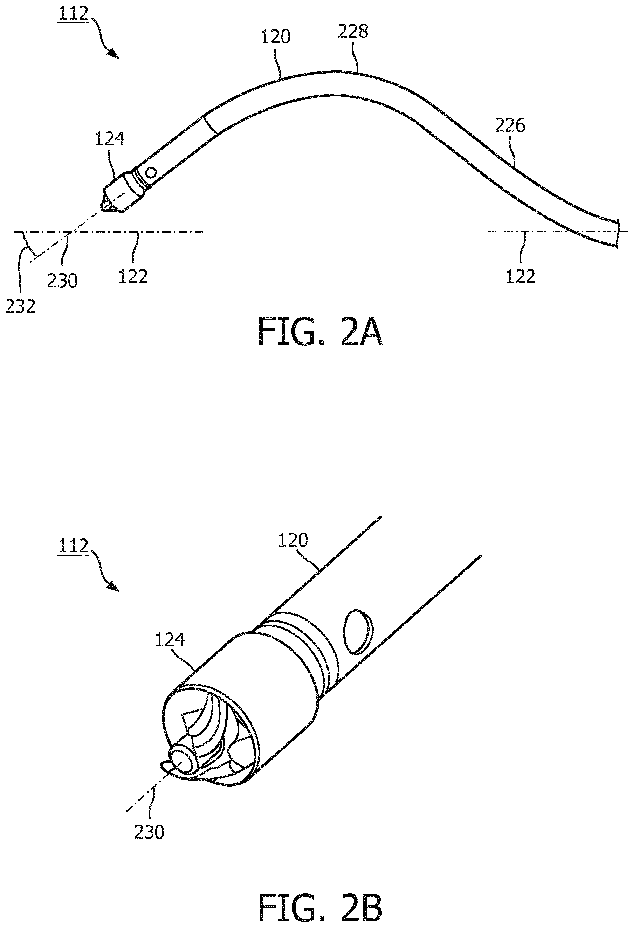

FIGS. 2A-2C illustrate the distal portion 112 of the catheter 108. The distal portion 120 of the outer sheath 116 is pre-shaped in a curved configuration or normally has a curved configuration ("normally" being understood as the catheter 108 not being subjected to any external contact forces due to, for example, contact with blood vessel walls) and is offset from the longitudinal axis 122. In some embodiments and as illustrated, the curved configuration of the distal portion 120 includes a double curve having a first, or proximal curve 226 and a second, or distal, curve 228. The proximal curve 226 bends or faces away from the longitudinal axis 122 and the distal curve 228 bends or faces toward the longitudinal axis 122. In some embodiments, the curved configuration causes a rotation axis 230 of the cutter assembly 124 to be disposed at an acute angle 232 relative to the longitudinal axis 122 (for example, about 45 degrees, although other angles may alternatively be used, such as greater than zero and less than 50 degrees, more particularly about 5 degrees, 10 degrees, 15 degrees, 20 degrees, 25 degrees, 30 degrees, 35 degrees, 40 degrees, 50 degrees, 55 degrees, 60 degrees, 65 degrees, 70 degrees, 75 degrees, 80 degrees, 85 degrees, or 90 degrees) (as used herein regarding angles, the term "about" being understood as the specified angle ± 10 percent). In some embodiments and situations, such dispositions of the cutter rotation axis 230 relative to the longitudinal axis 122 permit the cutter assembly 124 to oppose (that is, at least partially face toward) a blood vessel wall. Accordingly, the cutter assembly 124 can treat relatively large areas of plaque and provide relatively high luminal gain upon rotating the catheter 108 about the catheter rotation axis 110. Further, in some embodiments and situations, the double curve provides the catheter 108 with three contact points against the vasculature of the subject. More specifically, the proximal curve 226, the distal curve 228, and the cutter assembly 124 may each contact the vasculature of the subject.

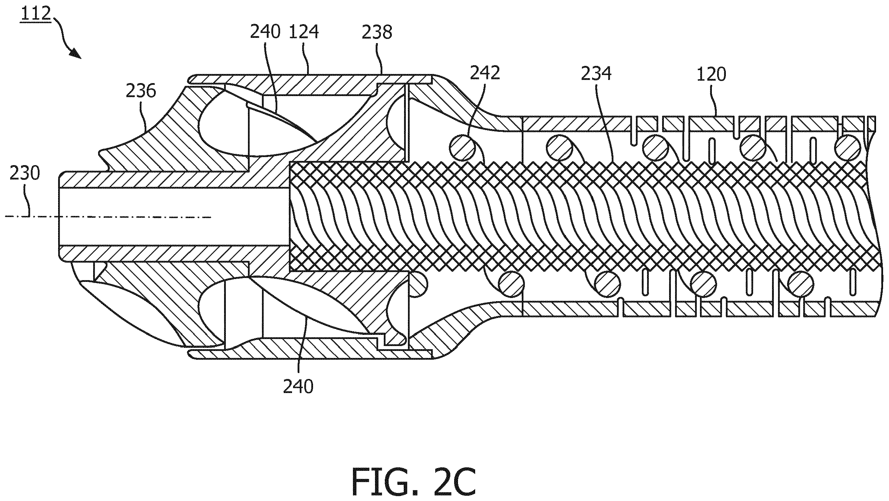

Referring specifically to FIG. 2C, a transverse sectional view of the distal portion 112 of the catheter 108 is provided. As illustrated, the outer sheath 116 carries a rotatable drive shaft 234 that couples the cutter assembly 124 to a prime mover (for example, a motor carried by the handle 106 - not shown). More specifically, the prime mover may rotate the drive shaft 234, which may in turn rotate a cutter 236 of the cutter assembly 124 with a cutter housing 238 of the cutter assembly 124 and about the cutter rotation axis 230. Rotation of the cutter 236 of causes one or more cutter elements 240 (for example, blades) to cut occlusive material and convey the occlusive material into the cutter housing 238 (a process also referred to as "debulking"). The drive shaft 234 also translatably fixes the cutter 236 relative to the outer sheath 116. In some embodiments, the cutter assembly 124 captures the cut occlusive material from the blood without the use of vacuum aspiration. In other embodiments, vacuum aspiration may assist capture of the cut occlusive material.

With continued reference to FIG. 2C, in some embodiments the atherectomy device 102 also includes an internal conveyor 242 that is coupled to and rotates with the drive shaft 234. As occlusive material is conveyed into the cutter housing 238 by the cutter 236, the conveyor 242 displaces the cut occlusive material proximally through the catheter 108 for discharge outside the subject's body. In some embodiments, this conveyance may occur without the use of vacuum aspiration assistance. In other embodiments, vacuum aspiration may assist conveyance of the cut occlusive material.

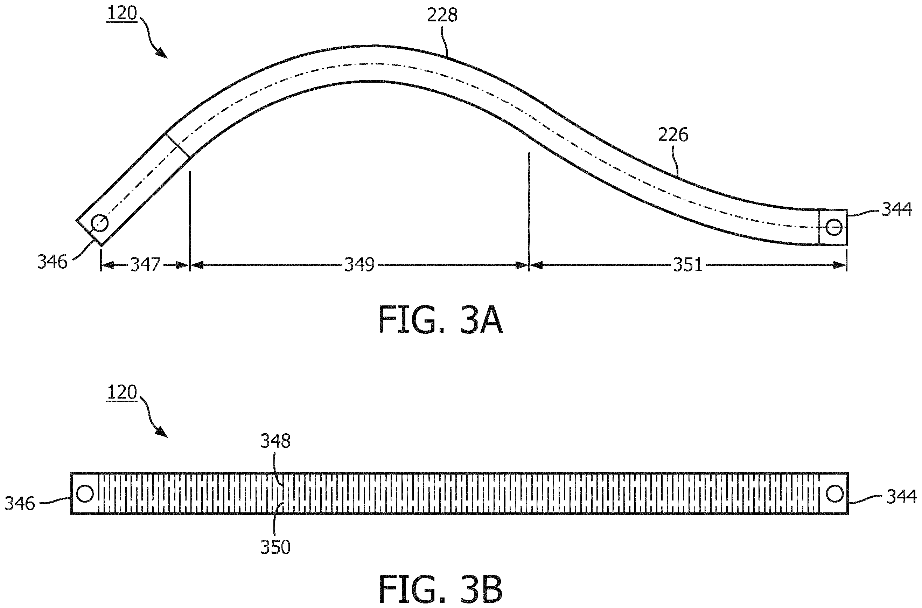

FIGS. 3A-3B illustrate the distal portion 120 of the outer sheath 116 of the catheter 108. In FIG. 3A, the distal portion 120 of the outer sheath 116 is illustrated in its normal curved configuration, and in FIG. 3B, the distal portion 120 of the outer sheath 116 is illustrated in a deflected configuration. In some embodiments, the distal portion 120 comprises one or more materials that are appropriate for being disposed within the vasculature of a subject, such as electropolished nitinol or polyether ether ketone (PEEK). In some embodiments, such materials and others may be set in, or urged to normally occupy, a curved configuration by constraining them in the curved configuration and applying thermal and/or electrical energy. In some embodiments, the distal portion 120 has an outer diameter of about 0.067 inches (that is, 0.067 inches ± 0.0008 inches) [about 1.7 mm (that is, 1.7 mm ± 0.02 mm)] and an inner diameter of about 0.059 inches (that is, 0.059 inches ± 0.0008 inches) [about 1.5 mm (that is, 1.5 mm ± 0.02 mm)].

In some embodiments and as illustrated in FIG. 3A, in the normal configuration the distal portion 120 of the outer sheath 116 has a length (that is, a distance between a proximal end 344 and a distal end 346, or a distance along the longitudinal axis 122 of the catheter 108) of about 1.549 inches (that is, 1.549 inches ± 0.0008 inches) [about 39.34 mm (that is, 39.34 mm ± 0.02 mm)]. In some embodiments and as illustrated in FIG. 3A, in the normal configuration the distal portion 120 of the outer sheath 116 has an offset dimension or a span (that is, a distance between the proximal end 344 and the distal end 346 and the second curve 228, or a distance perpendicular to the longitudinal axis 122 of the catheter 108) of about 0.395 inches (that is, 0.395 inches ± 0.020 inches) [about 10.03 mm (that is, 10.03 mm ± 0.51 mm)].

With continued reference to FIG.3A, the distal portion 120 includes a distal section 347, an intermediate section 349 (which defines the second curve 228 described above), and a proximal section 351 (which defines the first curve 226 described above). The distal section 347, the intermediate section 349, and the proximal section 351 may have about the dimensions shown in Table 1 (angles are relative to the longitudinal axis 122 of the catheter 108, distances are projected onto the longitudinal axis 122 of the catheter 108 - "about" being understood as the specified dimension ± 10 percent, and the term "substantially" being understood as the specified dimension ± percent).

**Table 1.**

| **Section** | **Angle (degrees)** | **Distance (in. [mm])** | **Radius (in. [mm])** |
|---|---|---|---|
| Distal (347) | 45 | 0.18 [4.6] | -- |
| Intermediate (349) | -- | 0.54 [14] | 0.48 [12] |
| Proximal (351) | -- | 1.23 [31.2] | 0.81 [21] |

The distal portion 120 of the outer sheath 116 may be relatively stiff to permit the catheter 108 to remove plaque from the vasculature in a curved configuration of the distal portion 120, and the distal portion 120 may be sufficiently flexible to deflect (for example and as shown in FIG. 3B, to a relatively straight configuration) while navigating the vasculature of a subject. To provide flexibility, in some embodiments the distal portion 120 includes a plurality of slits or kerfs 348 (that is, absences of material) that each extend through the wall of the distal portion 120. Such kerfs 348 may be provided by, for example, laser cutting processes. In some embodiments and as illustrated in FIG. 3B, the distal portion 120 includes first pattern of alternating kerfs 348 and non-slit or non-kerf portions 350 (that is, portions of the distal portion 120 include material and lacking kerfs 348) over its length. For example, the pattern of alternating kerfs 348 and non-kerf portions 350 may include, at each axial position within the pattern, a first kerf 348 having a longitudinal width of about 0.0015 inches [0.04 mm] and a circumferential length of about 90 degrees, a first non-kerf portion 350 having a circumferential length of about 30 degrees, a second kerf 348 having a longitudinal width of about 0.0015 inches [0.04 mm] and a circumferential length of about 90 degrees, a second non-kerf portion 350 having a circumferential length of about 30 degrees, a third kerf 348 having a longitudinal width of about 0.0015 inches [0.04 mm] and a circumferential length of about 90 degrees, and a third non-kerf portion 350 having a circumferential length of about 30 degrees (as used herein regarding kerf, non-kerf portion, and pattern dimensions, the term "about" being understood as the specified dimension ± 10 percent). Sets of kerfs 348 and non-kerf portions 350 in each axial position may be offset from sets of kerfs 348 and non-kerf portions 350 at one or more adjacent axial positions by (that is, the pattern may have a pitch of) about 0.012 inches [0.03 mm]. As illustrated, the pitch may be constant along the length of the outer sheath 116. In other embodiments, the pitch may be variable. As illustrated, the kerfs 348 may be perpendicular to the longitudinal axis (that is, the pattern may have a pitch angle of zero degrees). In other embodiments, the kerfs 348 may be non-perpendicular to the longitudinal axis (that is, the pattern may have a non-zero pitch angle). In some embodiments, the pattern may have a variable pitch angle.

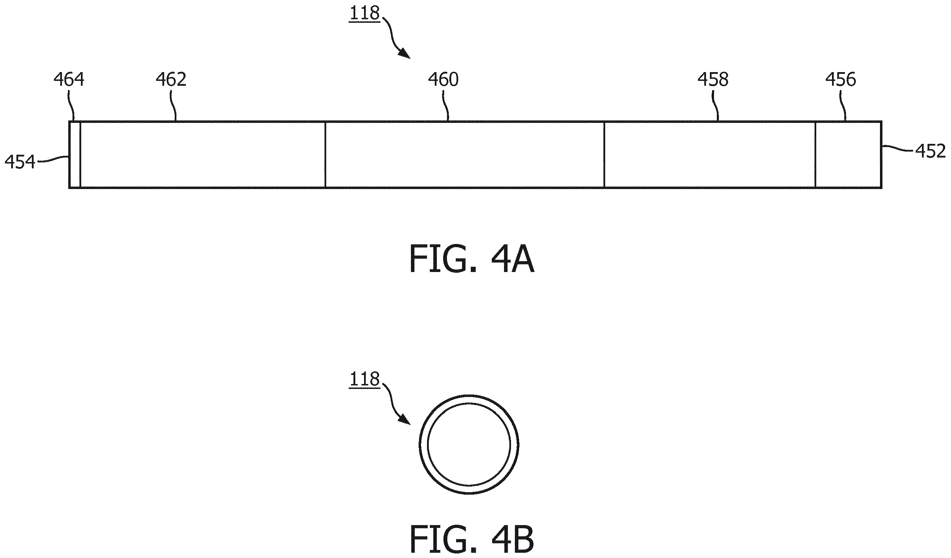

FIGS. 4A-4B illustrate the proximal portion 118 of the outer sheath 116 of the catheter 108. In some embodiments, the proximal portion 118 comprises one or more materials that are appropriate for being disposed within the vasculature of a subject, such as tempered stainless steel. In some embodiments, the proximal portion 118 has an outer diameter of about 0.061 inches (that is, 0.061 inches ± 0.0005 inches) [about 1.5 mm (that is, 1.5 mm ± 0.01 mm)] and an inner diameter of about 0.052 inches (that is, 0.052 inches ± 0.0005 inches) [about 1.3 mm (that is, 1.3 mm ± 0.01 mm)].

In some embodiments and as illustrated, the proximal portion 118 of the outer sheath 116 includes several sections having different flexibility characteristics. Generally, the proximal portion 118 is relatively stiff near its proximal end 452 to facilitate pushability of the catheter 108, and the proximal portion 118 is relatively flexible near its distal end 454 to facilitate navigating the vasculature of a subject. More specifically, the proximal portion 118 includes a relatively stiff proximal end section 456. In contrast to other sections, as described below, the proximal end section 456 lacks slits or kerfs. The proximal end section 456 may have a length of about 4.0 inches (that is, 4.0 inches ± 0.03 inches) [about 102 mm (that is, 102 mm ± 0.8 mm)].

The proximal portion 118 also includes a first intermediate section 458 that extends distally relative to the proximal end section 456. The first intermediate section 458 may have a length of about 9.9 inches (that is, 9.9 inches ± 0.03 inches) [about 251 mm (that is, 251 mm ± 0.8 mm)]. The first intermediate section 458 may be relatively stiff compared to other sections. More specifically, the first intermediate section 458 may include a second pattern of kerfs and non-kerf portions (not shown) over its length. For example, the pattern of alternating kerfs and non-kerf portions may include, at each axial position within the pattern, a first kerf having a longitudinal width of about 0.002 inches [about 0.05 mm] and a circumferential length of about 75 degrees, a first non-kerf portion having a circumferential length of about 15 degrees, a second kerf having a longitudinal width of about 0.002 inches [about 0.05 mm] and a circumferential length of about 75 degrees, a second non-kerf portion having a circumferential length of about 15 degrees, a third kerf having a longitudinal width of about 0.002 inches [about 0.05 mm] and a circumferential length of about 75 degrees, a third non-kerf portion having a circumferential length of about 15 degrees, a fourth kerf having a longitudinal width of about 0.002 inches [about 0.05 mm] and a circumferential length of about 75 degrees, and a fourth non-kerf portion having a circumferential length of about 15 degrees. Sets of kerfs and non-kerf portions in each axial position may be offset from sets of kerfs and non-kerfs portions at one or more adjacent axial positions by (that is, the pattern may have a pitch of) about 0.014 inches (that is, 0.014 inches ± 0.002 inches) [about 0.36 mm (that is, 0.36 mm ± 0.05 mm)]. The pitch may be constant or variable. The kerfs may be perpendicular to the longitudinal axis or non-perpendicular to the longitudinal axis. The pattern may have a variable pitch angle.

The proximal portion 118 also includes a second intermediate section 460 that extends distally relative to the first intermediate section 458. The second intermediate section 460 may have a length of about 25.0 inches (that is, 25.0 inches ± 0.03 inches) [about 635 mm (that is, 635 mm ± 0.8 mm)]. The second intermediate section 460 may be relatively flexible compared to the first intermediate section 458. More specifically, the second intermediate section 460 may include a third pattern of kerfs and non-kerf portions (not shown) over its length. For example, the pattern of alternating kerfs and non-kerf portions may include, at each axial position within the pattern, a first kerf having a longitudinal width of about 0.002 inches [about 0.05 mm] and a circumferential length of about 75 degrees, a first non-kerf portion having a circumferential length of about 15 degrees, a second kerf having a longitudinal width of about 0.002 inches [about 0.05 mm] and a circumferential length of about 75 degrees, a second non-kerf portion having a circumferential length of about 15 degrees, a third kerf having a longitudinal width of about 0.002 inches [about 0.05 mm] and a circumferential length of about 75 degrees, a third non-kerf portion having a circumferential length of about 15 degrees, a fourth kerf having a longitudinal width of about 0.002 inches [about 0.05 mm] and a circumferential length of about 75 degrees, and a fourth non-kerf portion having a circumferential length of about 15 degrees. Sets of kerfs and non-kerf portions in each axial position may be offset from sets of kerfs and non-kerfs portions at one or more adjacent axial positions by (that is, the pattern may have a pitch of) about 0.012 inches (that is, 0.012 inches ± 0.002 inches) [about 0.30 mm (that is, 0.30 mm ± 0.05 mm)]. The pitch may be constant or variable. The kerfs may be perpendicular to the longitudinal axis or non-perpendicular to the longitudinal axis. The pattern may have a variable pitch angle.

The proximal portion 118 also includes a third intermediate section 462 that extends distally relative to the second intermediate section 460. The third intermediate section 462 may have a length of about 16.0 inches (that is, 16.0 inches ± 0.03 inches) [about 406 mm (that is, 406 mm ± 0.8 mm)]. The third intermediate section 462 may be relatively flexible compared to the second intermediate section 460. More specifically, the third intermediate section 462 may include a fourth pattern of kerfs and non-kerf portions (not shown) over its length. For example, the pattern of alternating kerfs and non-kerf portions may include, at each axial position within the pattern, a first kerf having a longitudinal width of about 0.002 inches [about 0.05 mm] and a circumferential length of about 75 degrees, a first non-kerf portion having a circumferential length of about 15 degrees, a second kerf having a longitudinal width of about 0.002 inches [about 0.05 mm] and a circumferential length of about 75 degrees, a second non-kerf portion having a circumferential length of about 15 degrees, a third kerf having a longitudinal width of about 0.002 inches [about 0.05 mm] and a circumferential length of about 75 degrees, a third non-kerf portion having a circumferential length of about 15 degrees, a fourth kerf having a longitudinal width of about

0.002 inches [about 0.05 mm] and a circumferential length of about 75 degrees, and a fourth non-kerf portion having a circumferential length of about 15 degrees. Sets of kerfs and non-kerf portions in each axial position may be offset from sets of kerfs and non-kerfs portions at one or more adjacent axial positions by (that is, the pattern may have a pitch of) about 0.011 inches (that is, 0.011 inches ± 0.002 inches) [about 0.28 mm (that is, 0.28 mm ± 0.05 mm)]. The pitch may be constant or variable. The kerfs may be perpendicular to the longitudinal axis or non-perpendicular to the longitudinal axis. The pattern may have a variable pitch angle.

The proximal portion 118 further includes a relatively stiff distal end section 464. The distal end section 464 lacks slits or kerfs. The distal end section 464 may have a length of about 0.030 inches (that is, 0.030 inches ± 0.002 inches) [about 0.76 mm (that is, 0.76 mm ± 0.05 mm)].

In some embodiments and as illustrated, the atherectomy system 100 lacks any components for actively deflecting or reconfiguring the catheter 108 (for example, pull wires for deflecting or reconfiguring the catheter 108). Stated another way, in some embodiments the catheter 108 is only passively deflected or reconfigured (that is, reconfiguration or defection caused by external contact forces due to, for example, extending through an introducer sheath and/or contact with blood vessel walls). In some embodiments, the lack of components for actively deflecting or reconfiguring the catheter 108 permits the catheter 108 to have a relatively small overall size, such as 6-French. In some embodiments, the catheter 108 may have other overall sizes, such as 7-French, 8-French. 9-French, or the like.

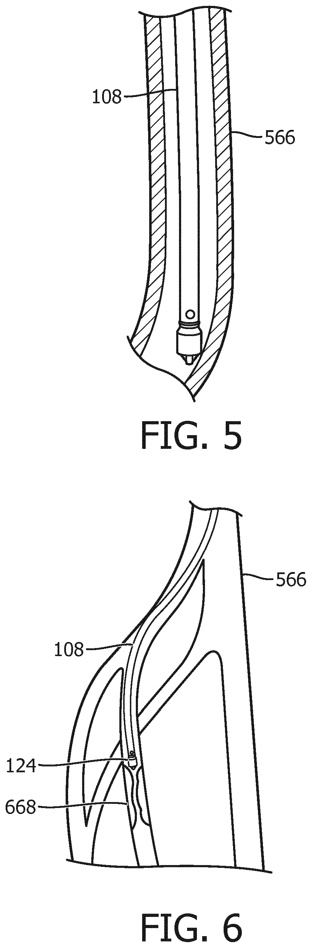

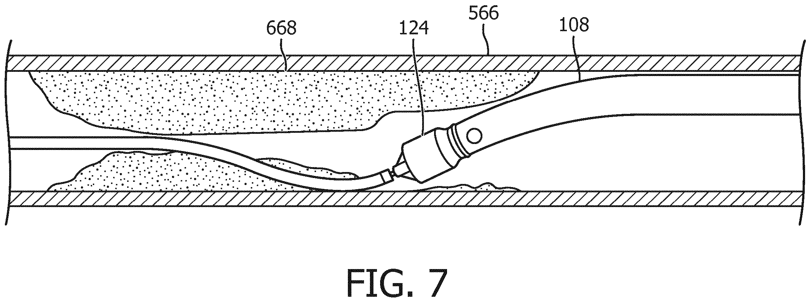

FIGS. 5-7 illustrate the catheter 108 navigating the vasculature 566 of a subject and removing plaque. FIG. 5 illustrates the catheter 108 being passively deflected to a relatively straight configuration while navigating the vasculature 566. As described above, the catheter 108 may be passively deflected or reconfigured by extending through an introducer sheath and/or contacting blood vessel walls. FIG. 6 illustrates the catheter 108 occupying a curved configuration and the cutter assembly 124 being disposed near plaque 668 within the vasculature 566. FIG. 7 illustrates the catheter 108 occupying a curved configuration and the cutter assembly 124 removing plaque 668 from the vasculature 566. The catheter 108 may be rotated about the catheter rotation axis 110 (or "swept") to further remove plaque 668 and provide relatively high luminal gain.

Although it is not shown in the drawings, when the distal portion 120 of the outer sheath 116 of the catheter 108 is inserted within a lumen of an introducer sheath, passing through the lumen of the introducer sheath, and/or exiting the introducer sheath, the distal portion 120 and/or the cutter assembly 124 is designed to contact the inner wall of the introducer sheath. That is, the distal portion 120 of the outer sheath 116 is pre-shaped in a curved configuration or normally has a curved configuration to ensure that the distal portion 120 and/or the cutter assembly 124 always contacts the inner wall of the introducer sheath as the distal portion 120 and/or the cutter assembly 124 pass through the lumen formed within the introducer sheath. Similarly, as the distal portion 120 and/or the cutter assembly 124 exits the introducer sheath, the distal portion 120 and/or the cutter assembly 124 contacts vasculature 566 and/or the plaque 668.

The foregoing discussion has been presented for purposes of illustration and description. The foregoing is not intended to limit the disclosure to the form or forms disclosed herein. In the foregoing Summary for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method disclosed is not to be interpreted as reflecting an intention that the claims require more features than are expressly recited in each claim. Rather, the scope of the invention is defined by the appended claims.

Moreover, though the description has included description of one or more aspects, embodiments, and/or configurations and certain variations and modifications, other variations, combinations, and modifications are within the scope of the disclosure, for example, as may be within the skill and knowledge of those in the art, after understanding the present disclosure

## Claims

1. An atherectomy device (102), comprising:
a handle (106) configured to be manipulated by a user;
a catheter comprising:
an outer sheath (116) comprising:
a proximal portion (118) coupled to and extending distally relative to the handle (106), the proximal portion (118) defining a longitudinal axis of the catheter;
a distal portion (120) coupled to and extending distally relative to the proximal portion (118), the distal portion (120) normally having a curved configuration and being offset from the longitudinal axis (122), and the distal portion (120) comprising a pattern of slits and non-slit portions or a pattern of kerfs and non-kerf portions;
a drive shaft carried within and rotatable relative to the outer sheath (116); and
a cutter assembly (124) coupled to and extending distally relative to the distal portion (120) of the outer sheath (116), the cutter assembly (124) including a cutter being translatably fixed relative to the outer sheath (116), the cutter coupled to the drive shaft and rotated about a cutter rotation axis upon rotation of the drive shaft relative to the outer sheath (116), the cutter rotation axis normally being disposed at an acute angle (232) relative to the longitudinal axis (122);
wherein the curved configuration comprises a proximal curve (226) and distal curve (228); and
wherein the proximal curve (226) bends away from the longitudinal axis (122) and the distal curve (228) bends toward the longitudinal axis (122).

2. The atherectomy device (102) of claim 1, wherein the catheter is rotatable about a catheter rotation axis relative to the handle (106).

3. The atherectomy device (102) of claim 2, wherein the catheter rotation axis is collinear with the longitudinal axis (122).

4. The atherectomy device (102) of claim 1, wherein the distal portion (120) comprises a proximal section (351) bending away from the longitudinal axis (122), an intermediate section (462) disposed distally of the proximal section (351) and bending towards the longitudinal axis (122), and a distal section (347) disposed distally of the intermediate section (462), the distal section (347) extending linearly and disposed at the acute angle relative to the longitudinal axis (122).

5. The atherectomy device (102) of claim 4, wherein the proximal section (351) defines the proximal curve (226), and the proximal curve (226) has a radius of 0.81 inches ± 10% , which is 21 mm ± 10%, and a length projected onto the longitudinal axis (122) of 31.2 millimeters ± 10%.

6. The atherectomy device (102) of claim 4, wherein the intermediate section (462) defines the distal curve (228), and the distal curve (228) has a radius of 0.48 inches ± 10%, which is 12 mm ± 10%, and a length projected onto the longitudinal axis (122) of 14 millimeters ± 10%.

7. The atherectomy device (102) of claim 4, wherein the distal section (347) has a length projected onto the longitudinal axis (122) of 4.6 millimeters ± 10%.

8. The atherectomy device (102) of claim 1, wherein the pattern is a first pattern, and wherein the proximal portion (118) comprises a second pattern, which is a pattern of slits and non-slit portions or a pattern of kerfs and non-kerf portions, the second pattern being different than the first pattern.

9. The atherectomy device (102) of claim 1, wherein the acute angle (232) is 45 degrees ± 10%.

## Patentansprüche

1. Atherektomievorrichtung (102), umfassend:
einen Griff (106), der konfiguriert ist, um von einem Benutzer gehandhabt zu werden;
einen Katheter, umfassend:
eine äußere Hülle (116), umfassend:
einen proximalen Abschnitt (118), der an den Griff (106) gekoppelt ist und sich in Bezug dazu distal erstreckt, wobei der proximale Abschnitt (118) eine Längsachse des Katheters definiert;
einen distalen Abschnitt (120), der an den proximalen Abschnitt (118) gekoppelt ist und sich in Bezug dazu distal erstreckt, wobei der distale Abschnitt (120) normalerweise eine gekrümmte Konfiguration aufweist und von der Längsachse (122) versetzt ist, und wobei der distale Abschnitt (120) ein Muster aus Abschnitten mit Schlitzen und ohne Schlitze oder ein Muster aus Abschnitten mit Kerben und ohne Kerben umfasst;
eine Antriebswelle, die innerhalb der äußeren Hülle (116) getragen wird und in Bezug dazu drehbar ist; und
eine Schneideranordnung (124), die an den distalen Abschnitt (120) der äußeren Hülle (116) gekoppelt ist und sich in Bezug dazu distal erstreckt, wobei die Schneideranordnung (124) einen Schneider beinhaltet, der in Bezug zur äußeren Hülle (116) verschiebbar befestigt ist, wobei der Schneider an die Antriebswelle gekoppelt ist und sich bei Drehung der Antriebswelle in Bezug zur äußeren Hülle (116) um eine Schneiderdrehachse dreht, wobei die Schneiderdrehachse normalerweise in einem spitzen Winkel (232) in Bezug zur Längsachse (122) angeordnet ist;
wobei die gekrümmte Konfiguration eine proximale Krümmung (226) und eine distale Krümmung (228) umfasst; und
wobei sich die proximale Krümmung (226) von der Längsachse (122) weg biegt und die distale Krümmung (228) sich zur Längsachse (122) hin biegt.

2. Atherektomievorrichtung (102) nach Anspruch 1, wobei der Katheter in Bezug zum Griff (106) um eine Katheterdrehachse drehbar ist.

3. Atherektomievorrichtung (102) nach Anspruch 2, wobei die Katheterdrehachse kollinear mit der Längsachse (122) ist.

4. Atherektomievorrichtung (102) nach Anspruch 1, wobei der distale Abschnitt (120) einen proximalen Teilabschnitt (351) umfasst, der sich von der Längsachse (122) weg biegt, einen mittleren Teilabschnitt (462), der distal von dem proximalen Teilabschnitt (351) angeordnet ist und sich in Richtung der Längsachse (122) biegt, und einen distalen Teilabschnitt (347), der distal von dem mittleren Teilabschnitt (462) angeordnet ist, wobei sich der distale Teilabschnitt (347) linear erstreckt und in einem spitzen Winkel in Bezug zur Längsachse (122) angeordnet ist.

5. Atherektomievorrichtung (102) nach Anspruch 4, wobei der proximale Teilabschnitt (351) die proximale Krümmung (226) definiert und die proximale Krümmung (226) einen Radius von 0,81 Zoll ± 10 % aufweist, was 21 mm ± 10% entspricht, und eine auf die Längsachse (122) projizierte Länge von 31,2 Millimeter ± 10%.

6. Atherektomievorrichtung (102) nach Anspruch 4, wobei der mittlere Teilabschnitt (462) die distale Krümmung (228) definiert und die distale Krümmung (228) einen Radius von 0,48 Zoll ± 10% aufweist, was 12 mm ± 10% entspricht, und eine auf die Längsachse (122) projizierte Länge von 14 Millimeter ± 10%.

7. Atherektomievorrichtung (102) nach Anspruch 4, wobei der distale Teilabschnitt (347) eine auf die Längsachse (122) projizierte Länge von 4,6 Millimetern ± 10% aufweist.

8. Atherektomievorrichtung (102) nach Anspruch 1, wobei das Muster ein erstes Muster ist und wobei der proximale Abschnitt (118) ein zweites Muster umfasst, das ein Muster aus Abschnitten mit Schlitzen und ohne Schlitze oder ein Muster aus Abschnitten mit Kerben und ohne Kerben ist, wobei sich das zweite Muster von dem ersten Muster unterscheidet.

9. Atherektomievorrichtung (102) nach Anspruch 1, wobei der spitze Winkel (232) 45 Grad ± 10% beträgt.

## Revendications

1. Dispositif d'athérectomie (102), comprenant :
une poignée (106) configurée pour être manipulée par un utilisateur ;
un cathéter, comprenant :
une gaine extérieure (116) comprenant :
une partie proximale (118) couplée à la poignée (106) et s'étendant de manière distale par rapport à celle-ci, la partie proximale (118) définissant un axe longitudinal du cathéter ;
une partie distale (120) couplée à la partie proximale (118) et s'étendant de manière distale par rapport à celle-ci, la partie distale (120) présentant normalement une configuration incurvée et étant décalée par rapport à l'axe longitudinal (122), et la partie distale (120) comprenant un motif de fentes et de parties non-fendues ou un motif d'entailles et de parties non-entaillées ;
un arbre d'entraînement porté à l'intérieur de la gaine extérieure (116) et pouvant tourner par rapport à celle-ci ; et
un ensemble de dispositif de coupe (124) couplé à et s'étendant distalement par rapport à la partie distale (120) de la gaine extérieure (116), l'ensemble de dispositif de coupe (124) incluant un dispositif de coupe fixé pouvant être déplacé en translation par rapport à la gaine extérieure (116), le dispositif de coupe étant couplé à l'arbre d'entraînement et tourné autour d'un axe de rotation de dispositif de coupe lors d'une rotation de l'arbre d'entraînement par rapport à la gaine extérieure (116), l'axe de rotation de dispositif de coupe étant normalement disposé à un angle aigu (232) par rapport à l'axe longitudinal (122) ;
dans lequel la configuration incurvée comprend une courbe proximale (226) et une courbe distale (228) ; et
dans lequel la courbe proximale (226) se courbe en s'éloignant de l'axe longitudinal (122) et la courbe distale (228) se courbe en direction de l'axe longitudinal (122).

2. Dispositif d'athérectomie (102) selon la revendication 1, dans lequel le cathéter peut tourner autour d'un axe de rotation de cathéter par rapport à la poignée (106).

3. Dispositif d'athérectomie (102) selon la revendication 2, dans lequel l'axe de rotation de cathéter est colinéaire à l'axe longitudinal (122).

4. Dispositif d'athérectomie (102) selon la revendication 1, dans lequel la partie distale (120) comprend une section proximale (351) se courbant à l'opposé de l'axe longitudinal (122), une section intermédiaire (462) disposée distalement par rapport à la section proximale (351) et se courbant vers l'axe longitudinal (122), et une section distale (347) disposée distalement par rapport à la section intermédiaire (462), la section distale (347) s'étendant linéairement et étant disposée au niveau de l'angle aigu par rapport à l'axe longitudinal (122).

5. Dispositif d'athérectomie (102) selon la revendication 4, dans lequel la section proximale (351) définit la courbe proximale (226), et la courbe proximale (226) présente un rayon de 0,81 pouce ± 10 %, soit 21 mm ± 10 %, et une longueur projetée sur l'axe longitudinal (122) de 31,2 millimètres ± 10 %.

6. Dispositif d'athérectomie (102) selon la revendication 4, dans lequel la section intermédiaire (462) définit la courbe distale (228), et la courbe distale (228) présente un rayon de 0,48 pouce ± 10 %, qui est de 12 mm ± 10 %, et une longueur projetée sur l'axe longitudinal (122) de 14 millimètres ± 10 %.

7. Dispositif d'athérectomie (102) selon la revendication 4, dans lequel la section distale (347) présente une longueur projetée sur l'axe longitudinal (122) de 4,6 millimètres ± 10 %.

8. Dispositif d'athérectomie (102) selon la revendication 1, dans lequel le motif est un premier motif, et dans lequel la partie proximale (118) comprend un second motif, qui est un motif de fentes et de parties non-fendues ou un motif d'entailles et de parties non-entaillées, le second motif étant différent du premier motif.

9. Dispositif d'athérectomie (102) selon la revendication 1, dans lequel l'angle aigu (232) est de 45 degrés ± 10 %.
